(19) Europäisches Patentamt
European Patent Office
Office européen des brevets

(11) Veröffentlichungsnummer : **0 182 173**
**B1**

(12) # EUROPÄISCHE PATENTSCHRIFT

(45) Veröffentlichungstag der Patentschrift : **13.12.89**

(51) Int. Cl.[4] : **C 07 C147/12**// C09B62/507

(21) Anmeldenummer : **85113960.0**

(22) Anmeldetag : **02.11.85**

(54) **Monocyclische Bis-oxethylsulfonyl-aniline und Verfahren zu ihrer Herstellung.**

(30) Priorität : **12.11.84 DE 3441273**

(43) Veröffentlichungstag der Anmeldung : **28.05.86 Patentblatt 86/22**

(45) Bekanntmachung des Hinweises auf die Patenterteilung : **13.12.89 Patentblatt 89/50**

(84) Benannte Vertragsstaaten : **CH DE FR GB IT LI**

(56) Entgegenhaltungen :
**EP-A- 0 181 585**
**DE-A- 2 100 080**

(73) Patentinhaber : **HOECHST AKTIENGESELLSCHAFT**
**Postfach 80 03 20**
**D-6230 Frankfurt am Main 80 (DE)**

(72) Erfinder : **Papenfuhs, Theodor, Dr.**
**Heinrich-Bleicher-Strasse 40**
**D-6000 Frankfurt am Main 50 (DE)**
Erfinder : **König, Gerd, Dr.**
**Breckenheimer Strasse 32**
**D-6238 Hofheim am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft neue monocyclische Bis-oxethylsulfonyl-aniline der allgemeinen Formel (1)

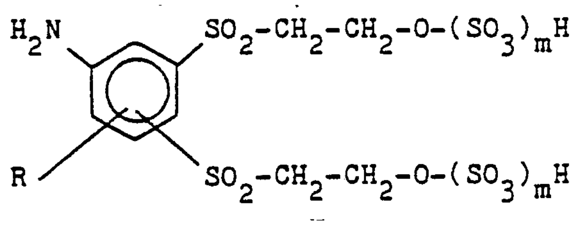

(1)

in welcher R ein Wasserstoff-, Chlor- oder Bromatom oder eine $Alkyl_{C_1-C_4}$- oder $Alkoxy_{C_1-C_4}$-Gruppe bedeutet und m die Zahl 0 oder 1 darstellt, und in welcher die Oxethylsulfonyl(schwefelsäurehalbester)-Gruppen in o- oder p-Stellung zueinander stehen, sowie Verfahren zu ihrer Herstellung, indem man eine Verbindung der Formel (2)

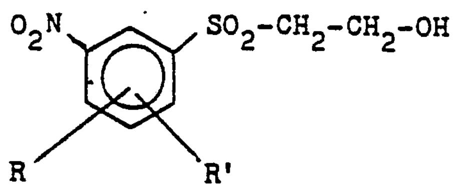

(2)

in welcher R die vorstehend genannte Bedeutung hat, R' ein Chlor- oder Bromatom bedeutet und in o- oder p-Stellung zur β-Oxethylsulfonyl-Gruppe steht, gegebenenfalls unter Zusatz von Wasser als Verdünnungsmittel, mit der mindestens stöchiometrischen Menge Thioglykol in Gegenwart einer mindestens stöchiometrischen Menge einer säurebindenden Alkalimetall- oder Erdalkalimetallverbindung bei Temperaturen von 10-60 °C, vorzugsweise 20-40 °C, umsetzt, die so erhaltene Verbindung der Formel (3)

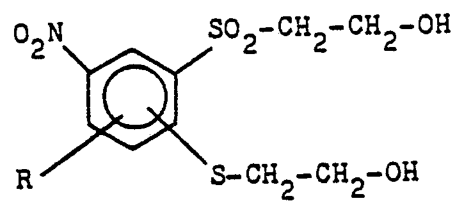

(3)

in welcher R die vorstehend genannte Bedeutung hat und die beiden schwefelhaltigen Substituenten in o- oder p-Stellung zueinander stehen, ohne oder nach erfolgter Zwischenisolierung, nach Zugabe katalytischer Mengen von Wolframtrioxid oder Alkalimetallwolframat und ggf. Wasser und Einstellung eines pH-Werts von 4-6 durch Zugabe einer nichtoxydierenden anorganischen oder organischen Säure, mittels Wasserstoffperoxid bei Temperaturen von 40 °C bis 100 °C oxidiert und die hierbei erhaltene Verbindung der Formel (4)

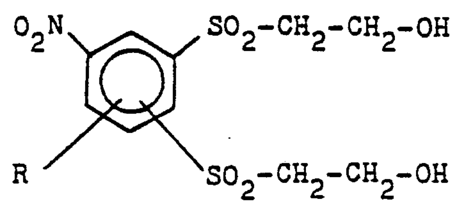

(4)

in welcher R die vorstehend genannte Bedeutung hat und die beiden β-Oxethylsulfonyl-Gruppen in o- oder p-Stellung zueinander stehen, in an sich bekannter Weise mittels Eisen/Säure oder katalytisch mit Nickel- oder Edel-metallkatalysatoren in wäßrigem Medium reduziert und die hierbei erhaltenen Verbindungen der allgemeinen Formel (5)

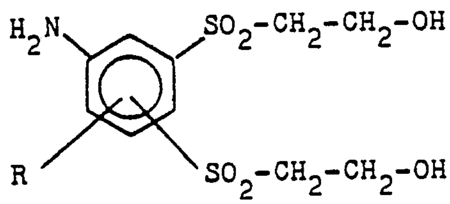

(5)

in welcher R die vorstehend genannte Bedeutung hat und die beiden β-Oxethylsulfonyl-Gruppen in o- oder p-Stellung zueinanderstehen, gegebenenfalls in an sich bekannter Weise mit konzentrierter Schwefelsäure, Oleum, einer Mischung aus Schwefelsäure-Monohydrat und Oleum, Schwefeltrioxid oder Chlorsulfonsäure in den Bis-schwefelsäurehalbester der genannten Formel (1) mit m = 1 überführt.

Zur Verfahrensdurchführung sei im einzelnen folgendes ausgeführt:

Bei der ersten Stufe (Kondensationsreaktion) ist es zweckmäßig, die Ausgangsverbindung der Formel (2) mit einem Überschuß von 10-50 Molprozent an Thioglykol umzusetzen. Der eventuelle Zusatz an Wasser und dessen Menge ist auf eine ausreichende Rührbarkeit der Reaktionsmischung in dieser Stufe und in den sich anschließenden Stufen abzustellen. Nach Zugabe des Thioglykols und eventuell von Wasser bei Temperaturen von 10-60 °C, vorzugsweise 20-40 °C, trägt man in die Mischung im Verlauf von 1-5 Stunden, vorzugsweise 2 bis 3 Stunden, anteilweise eine mindestens stöchiometrische Menge einer säurebindenden Alkalimetall- oder Erdalkalimetallverbindung, wie beispielsweise ein Alkalimetall- oder Erdalkalimetalloxid, -hydroxid oder -carbonat, vorzugsweise Natrium- oder insbesondere Kaliumcarbonat oder Magnesiumoxid, ein. Anschließend wird bis zur Beendigung der Umsetzung (Kontrolle erfolgt durch Dünnschichtchromatografie oder HPLC) (HPLC = High performance liquid chromatography analysis) nachgerührt (3-10 Stunden) und dann durch Zulauf von Säure (Mineral- oder Essigsäure) ein pH-Wert von 4-6 eingestellt. Das erhaltene Oxethylsulfonyl-nitrophenyl-oxethylsulfid der Formel (3) kann nach Abkühlen auf Raumtemperatur durch Filtration in hoher Ausbeute isoliert werden.

Vorteilhafter ist es jedoch, die erhaltene Suspension der genannten Verbindung direkt nach Zusatz katalytischer Mengen Wolframtrioxid oder Alkalimetallwolframat (1-10 g pro Mol Verbindung der Formel (3)) mit der mindestens stöchiometrischen Menge Wasserstoffperoxid (2 Mol pro Mol Verbindung der Formel (3)) zu versetzen und der Oxidation (zweite Verfahrensstufe) zu unterwerfen und nach beendeter Oxidation (Kontrolle durch Dünnschichtchromatografie oder HPLC) das gebildete Oxidationsprodukt (Bis-ß-oxethylsulfon der Formel (4)) aus der angefallenen Reaktionsmischung durch Kühlen auf 0 bis 20 °C auszukristallisieren und anschließend durch Filtration zu isolieren.

Die darauffolgende Reduktion der Nitrogruppe (dritte Verfahrensstufe) kann nach bekannten Methoden, beispielsweiee mittels Eisen/Säure oder katalytische Reduktion an Nickel- oder Edelmetall-Katalysatoren in wäßrigem Medium durchgeführt werden (HOUBEN-WEYL Bd. XI/1, S. 394-406/374-382) und liefert die entsprechenden Aminoverbindungen der Formel (1) mit m = 0 in ausgezeichneter Qualität und Ausbeute.

Selbstverständlich können die vorstehend genannten einzelnen Stufen des erfindungsgemäßen Verfahrens auch isoliert oder kombiniert in Gegenwart gängiger Lösungs- oder Verdünnungsmittel, sofern sie unter Reaktionsbedingungen ausreichend stabil sind, durchgeführt werden. Der Vorteil einer besonders einfachen Verfahrensdurchführung im rein wäßrigen System und damit in technisch üblichen Apparaten geht dadurch aber teilweise verloren (Erfordernis der Lösungsmittel-Filtration und/oder -Regeneration in speziellen Apparaturen, wodurch durch Lösungsmittel-Verluste zusätzliche Kosten auftreten, ohne daß Ausbeute und/oder Qualität positiv beeinflußt werden.) Eine solche Verfahrensweise stellt daher keine bevorzugte Ausführung dar.

Die sich ggfs. anschließende Veresterung der Verbindungen der Formel (1) mit m = 0 zu den Verbindungen der allgemeinen Formel (1) mit m = 1 erfolgt mittels konzentrierter Schwefelsäure, Oleum, einer Mischung aus Schwefelsäure-Monohydrat und Oleum, Schwefeltrioxid oder Chlorsulfonsäure nach bekannten Verfahren.

Weitere Einzelheiten der Durchführung des erfindungsgemäßen Herstellungsverfahrens und der Eigenschaften und physikalischen Daten der neuen Verbindungen der genannten allgemeinen Formel (1), sowie typische Reaktions- und Isolierparameter sind den Ausführungsbeispielen zu entnehmen. Die als Ausgangsverbindungen eingesetzten Verbindungen der genannten allgemeinen Formel (2) können auf die in der DE-PS 859 462 beschriebene Weise hergestellt werden.

Die neuen Bis-oxethylsulfonyl-aniline der genannten allgemeinen Formel (1) stellen wertvolle Vorprodukte zur Herstellung von Reaktivfarbstoffen der Vinylsulfonreihe dar. Durch die mit diesen Verbindungen in den daraus hergestellten Farbstoffen erreichbare Erhöhung faserreaktiver Zentren kann eine bessere Farbstoff-Fixierung auf der Cellulosefaser (Baumwolle) und damit eine höhere Farbstoffausbeute und eine geringere Abwasserbelastung durch inaktiven Farbstoff erzielt werden. Die neuen Vorprodukte können beispielsweise als Diazokomponenten bei der Herstellung von Reaktivazofarbstoffen der Vinylsulfonreihe eingesetzt werden.

Gegenüber reaktiven Azofarbstoffen, die mit den aus der EP-OS 0107614 bekannten 2,4-Bis-oxethylsulfonyl-anilinen (Diazokomponenten) hergestellt werden, zeichnen sich die auf Basis der erfindungsgemäßen Bis-oxethylsulfonyl-aniline hergestellten reaktiven Azofarbstoffe durch eine geringere Alkaliempfindlichkeit aus. Die neuen 2,3- 2,5- und 3,4-Bis-oxethylsulfonylaniline stellen somit hinsichtlich der Herstellungsmöglichkeit von reaktiven Azofarbstoffen mit verbesserten Eigenschaften einen erheblichen technischen Fortschritt dar.

Die nachfolgenden Beispiele sollen das Verfahren näher erläutern, ohne es darauf zu beschränken.

### Beispiel 1

Eine Mischung aus 531 Teilen 4-Chlor-3-nitrophenyl-oxethylsulfon, 236 Teilen Thioglykol und 200 Teilen Wasser wird auf 25-30 °C erwärmt und bei dieser Temperatur unter Rühren und Sauerstoffausschluß innerhalb von 3 Stunden in Anteilen mit 175 Teilen festem Kaliumcarbonat versetzt. Man rührt 6-8 Stunden nach, bis durch dünnschichtchromatografische Kontrolle kein Ausgangsprodukt mehr nachweisbar ist, und kühlt dann auf 10-15 °C ab, wobei das 2-Nitro-4-oxethylsulfonyl-phenyl-oxethylsulfid in gelben Kristallen ausfällt. Man isoliert es bei 10-15 °C durch Absaugen auf einer Nutsche und erhält nach Neutralwaschen und Trocknen im Vakuum bei 50-60 °C 565 Teile 2-Nitro-4-oxethylsulfonyl-phenyl-oxethylsulfid vom Schmelzpunkt 108-110 °C, was einer Ausbeute von 92,0 % der Theorie, bezogen auf 4-Chlor-3-nitro-phenyl-oxethylsulfon, entspricht.

Das Produkt ist chromatografisch rein und zeigt in der Elementaranalyse die seiner Konstitution entsprechenden Werte (berechnet : $NO_2$ : 15,0 % ; S : 20,85 % ; OH : 11,1 % ; Cl : 0,0 % ; gefunden : $NO_2$ : 15.0/14.8 % ; S : 21,0 % ; OH : 11,1/11,0 % ; Rest-Cl : 0,012 %). Verwendet man an Stelle von Kaliumcarbonat entsprechende Mengen Natriumcarbonat, so muß die Eintrag- und Nachrührzeit um ca. 50 % verlängert werden, um ein vergleichbares Ergebnis zu erhalten.

### Beispiel 2

Eine Mischung aus 531 Teilen 2-Chlor-5-nitrophenyl-oxethylsulfon, 240 Teilen Thioglykol und 400 Teilen Wasser wird bei Raumtemperatur unter Rühren und Sauerstoffausschluß innerhalb von 3 Stunden gleichmäßig mit 175 Teilen Kaliumcarbonat versetzt und anschließend 12-15 Stunden bei 25 °C nachgerührt, bis eine dünnschichtchromatografische Kontrolle vollständige Umsetzung anzeigt. Man kühlt auf 10-15 °C ab und saugt den gelben Niederschlag auf einer Nutsche ab. Nach Neutralwäsche und Trocknen bei 60-80 °C im Vakuum erhält man 571 Teile 4-Nitro-2-oxethylsulfonyl-phenyl-oxethyl-sulfid vom Schmelzpunkt 117-119 °C, was einer Ausbeute von 93,0 % der Theorie, bezogen auf 2-Chlor-5-nitrophenyl-oxethylsulfon, entspricht. Das Produkt ist chromatografisch ein und zeigt in der Elementaranalyse die seiner Konstitution entsprechenden Werte (ber. $NO_2$ : 15,0 % ; S : 20,85 % ; OH : 11,1 % ; Cl : 0,0 % ; gefunden : $NO_2$ : 14,7/14,9 % ; S : 20,9 % ; OH : 11,0/10,9 % ; Rest-Cl : 0,005 %).

### Beispiel 3

Ersetzt man in Beispiel 2 das 2-Chlor-5-nitrophenyl-oxethylsulfon durch eine aliquote Menge an 2-Chlor-3-nitrophenyl-oxethylsulfon und arbeitet im übrigen in der dort angegebenen Weise, so erhält man 550 Teile 6-Nitro-2-oxethylsulfonyl-phenyl-oxethylsulfid vom Schmelzpunkt 108-110 °C in etwas geringerer Qualität (Rest-Cl-Gehalt : 0,3-0,4 %), das sich als solches ohne Einschränkung zur Oxidation einsetzen läßt. Die Ausbeute, bezogen auf eingesetztes 2-Chlor-3-nitrophenyl-oxethylsulfon, beträgt 89,6 % der Theorie.

### Beispiel 4

Eine Mischung aus 307 Teilen 2-Nitro-4-oxethylsulfonylphenyl-oxethylsulfid, 4 Teilen Natriumwolframat-dihydrat und 1000 Teilen Wasser wird bei 60 °C mit 20 %iger Essigsäure auf pH 5-5,5 eingestellt und dann im Verlaufe von 30 Minuten mit 117 Teilen 30 %igem Wasserstoffperoxid versetzt, wobei gleichzeitig die Innentemperatur auf 80 °C gesteigert wird. Anschließend läßt man bei 80-90 °C innerhalb einer weiteren Stunde nochmals gleichmäßig 152 Teile 30 %iges Wasserstoffperoxid zutropfen, wobei eine klare, nahezu farblose Lösung entsteht. Man rührt 10 Stunden bei 90-95 °C nach (Kontrolle auf vollständige Umsetzung durch Dünnschichtchromatografie) und kühlt dann unter Rühren auf 10-15 °C ab. Die sich dabei abscheidenden Kristalle werden abgesaugt, mit Wasser 2 mal abgedeckt und im Vakuum bei 60-80 °C getrocknet.

Man erhält 307 Teile 2-Nitro-1,4-bis-oxethylsulfonyl-benzol vom Schmelzpunkt 156-158 °C, was einer Ausbeute von 93,5 % der Theorie, bezogen auf eingesetztes 2-Nitro-4-oxethylsulfonyl-phenyloxethylsulfid entspricht.

Die Verbindung ist chromatografisch rein und entspricht in der Elementaranalyse der angegebenen Konstitution (berechnet : $NO_2$ : 13,55 % ; S : 18,9 % ; gefunden : $NO_2$ : 13,4/13,7 % ; S : 18,8 %).

### Beispiel 5

Zu einer gerührten Vorlage aus 307 Teilen 4-Nitro-2-oxethylsulfonyl-phenyloxethylsulfid, 4,5 Teilen Kaliumwolframat und 1200 Teilen Wasser, die mit 2 n Salzsäure auf pH 4,5-5 eingestellt ist, werden in 1

Stunde bei 70-75 °C 100 Teile 35 %iges Wasserstoffperoxid getropft. Man heizt in 30 Minuten auf 90-95 °C und tropft weitere 110 Teile 35 %iges Wasserstoffperoxid in 90 Minuten zu, rührt 13-14 Stunden bei dieser Temperatur nach (Kontrolle auf vollständige Umsetzung durch HPLC), kühlt dann auf 20-25 °C ab und isoliert den ausgefallenen, gelblich-weißen Niederschlag durch Filtration auf einer Nutsche. Man deckt zweimal mit Wasser ab, saugt trocken und trocknet im Vakuum bei 60-70 °C. Es resultieren 325 Teile 4-Nitro-1,2-bis-oxethylsulfonyl-benzol vom Schmelzpunkt 204-206 °C, entsprechend einer Ausbeute von 95,8 % der Theorie, bezogen auf eingesetztes 4-Nitro-2-oxethylsulfonyl-phenyloxethylsulfid.

Die Verbindung ist chromatografisch rein und zeigt in der Elementaranalyse ihrer Konstitution entsprechende Werte (berechnet : $NO_2$ : 13,55 % ; S : 18,9 % ; gefunden : $NO_2$ : 13,7 % ; S : 18,7/19,0 %).

Wird die Oxidation in Abwesenheit des Wolfram-Katalysators, aber sonst in der angegebenen Weise durchgeführt, so ist eine viermal längere Reaktionszeit und ein um 10-15 % höherer Wasserstoffperoxid-Bedarf erforderlich. Qualität und Ausbeute werden dadurch nicht verändert.

### Beispiel 6

Eine Mischung aus 265,5 Teilen 2-Chlor-5-nitrophenyl-oxethylsulfon, 157 Teilen Thioglykol und 100 Teilen Wasser wird bei 20-25 °C unter Rühren und Sauerstoffausschluß in 5 Stunden portionsweise mit 34 Teilen Magnesiumoxid versetzt. Man rührt unter langsamer Temperatursteigerung auf 30 °C ca. 5-6 Stunden nach, bis kein Ausgangsprodukt mehr nachweisbar ist (Kontrolle durch Dünnschichtchromatografie), setzt 900 Teile Wasser und 4 Teile Natriumwolframat-dihydrat zu und stellt die resultierende Lösung mit 85 %iger Phosphorsäure auf pH 5-5,5 ein. Nachfolgend tropft man in 60 Minuten, bei 60 °C beginnend, 210 Teile 30 %iges Wasserstoffperoxid zu, wobei die Temperatur auf 75-80 °C ansteigen soll. Anschließend läßt man bei 80-90 °C innerhalb einer weiteren Stunde nochmals gleichmäßig 160 Teile 3 %iges Wasserstoffperoxid zutropfen, rührt 10-12 Stunden bei 90-95 °C nach (Kontrolle auf vollständige Umsetzung durch HPLC) und kühlt dann unter Rühren auf 10-15 °C ab. Das dabei ausgefallene Produkt wird abgesaugt, mit Wasser neutral und salzfrei gewaschen und im Vakuum bei 60-80 °C getrocknet.

Man erhält 306,5 Teile 4-Nitro-1,2-bis-oxethylsulfonylbenzol vom Schmelzpunkt 203-205 °C, was einer Ausbeute von 90,4 % der Theorie, bezogen auf eingesetztes 2-Chlor-5-nitrophenyl-oxethylsulfon entspricht.

### Beispiel 7

Eine Suspension, bestehend aus 310 Teilen 4-Brom-3-nitrophenyl-oxethylsulfon, 118 Teilen Thioglykol und 300 Teilen Wasser wird bei 15-25 °C unter Rühren und Sauerstoffausschluß in 4 Stunden portionsweise mit 82,5 Teilen Kaliumcarbonat versetzt. Man rührt 1-2 Stunden bei 20-25 °C nach (Kontrolle auf vollständige Umsetzung durch Dünnschichtchromatografie), verdünnt den Ansatz mit 800 Teilen Wasser, setzt 5 Teile Kaliumwolframat zu und stumpft mit 50 %iger Essigsäure auf pH 5-5,5 ab.

Bei 50-55 °C beginnend werden nun in 60 Minuten 130 Teile 35 %iges Wasserstoffperoxid zugetropft, wobei die Temperatur auf maximal 75 °C ansteigen darf. Anschließend wird eine Stunde bei 75 °C nachgerührt. Daraufhin werden in 90 Minuten bei 75 °C nochmals 120 Teile 35 %iges Wasserstoffperoxid zugetropft. Nach Erhöhung der Temperatur auf 90-95 °C wird bis zur vollständigen Oxidation (Kontrolle durch HPLC, Dauer ca. 14-15 Stunden) bei dieser Temperatur nachgerührt. Man läßt abkühlen, saugt den ausgefallenen Niederschlag bei 15-20 °C auf einer Nutsche ab, wäscht zweimal mit 200 Teilen Eiswasser und trocknet im Vakuum bei 50-65 °C.

Man erhält 301,4 Teile 2-Nitro-1,4-bis-oxethylsulfonylbenzol vom Schmelzpunkt 154-156 °C, was einer Ausbeute von 88,9 % der Theorie, bezogen auf 4-Brom-3-nitrophenyloxethylsulfon, entspricht.

### Beispiel 8

In 400 Teile Wasser werden 75 Teile Eisenpulver eingetragen, 1 Teil konz. Salzsäure zugesetzt und die Mischung auf 85 °C erwärmt. In diese Anschlämmung trägt man unter gutem Rühren innerhalb einer Stunde 169,5 Teile 2-Nitro-1,4-bis-oxethylsulfonylbenzol ein. Dabei hält sich die Temperatur ohne weiteres Heizen bei 85-95 °C. Anschließend wird bei dieser Temperatur 30 Minuten nachgerührt, mit wäßriger Natronlauge auf pH 8 gestellt und heiß abgesaugt. Beim Erkalten kristallisiert das Produkt aus. Man filtriert auf einer Nutsche unf trocknet im Vakuum bei 70 °C. Es werden 101 Teile (65,3 % der Theorie) 2,5-Bis-oxethylsulfonylanilin vom Schmelzpunkt 111-114 °C als feinkristallines hellgelbes Pulver erhalten, dessen Elementar-Analyse (C : 38,5 %, H : 4,7 %, N : 4,6 %, S : 20,8 %) mit der angegebenen Struktur (ber. : C : 38,8 %, H : 4,5 %, N : 4,5 %, S : 20,7 %) in Einklang steht.

### Beispiel 9

In 350 Teile Wasser werden 50 Teile Eisenpulver eingetragen, 1 Teil konz. Salzsäure zugesetzt und die Mischung auf 85 °C erwärmt. In diese Anschlämmung trägt man unter gutem Rühren innerhalb einer Stunde 111 Teile 4-Nitro-1,2-bis-oxethylsulfonyl-benzol ein. Dabei hält sich die Temperatur ohne weiteres Heizen bei 85-95 °C. Anschließend wird bei dieser Temperatur 30 Minuten nachgerührt, mit wäßriger

Natronlauge auf pH 8 gestellt und heiß abgesaugt. Beim Erkalten kristallisiert das Produkt aus. Man filtriert auf einer Nutsche und trocknet im Vakuum bei 70 °C. Es werden 85,5 Teile (84,5 % der Theorie) 3,4-Bis-oxethylsulfonylanilin vom Schmelzpunkt 156-157 °C als feinkristallines hellgelbes Pulver erhalten, dessen Analyse (C : 38,7 %, H : 4,6 %, N : 4,6 %) die erwarteten Werte (ber. C : 38,8 %, H : 4,8 %, N : 4,5 %) zeigt.

Beispiel 10

Zur Überführung in den Bis-sulfatoester werden 61,8 Teile 2,5-Bis-oxethylsulfonylanilin bei Raumtemperatur in eine Mischung aus 200 Teilen Schwefelsäure-Monohydrat und 25 Teilen Oleum 65 % eingetragen und 12 Stunden gerührt, bis sich eine klare Lösung gebildet hat. Anschließend wird die Reaktionsmischung bei 0 bis maximal 5 °C in 1000 Teile 25 %ige KCl-Lösung eingetropft, 3 Stunden nachgerührt, das Produkt abgesaugt, der Filterkuchen mit 25 %iger KCl-Lösung gewaschen und bei 40 °C im Vakuum getrocknet. Man erhält 159 g der Verbindung der Formel

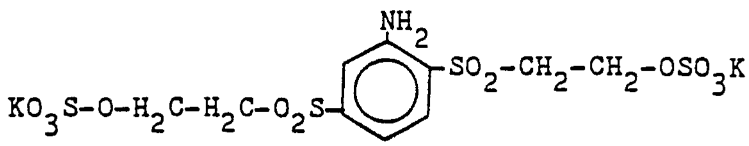

Der durch Diazotierung ermittelte Gehalt an Reinsubstanz beträgt 59,4 %. Die Substanz ist chromatographisch einheitlich.

Setzt man an Stelle von 2,5-Bis-oxethylsulfonyl-anilin das nach beispiel 9 hergestellte 3,4-Isomere ein und arbeitet im übrigen in der vorstehend angegebenen Weise, so erhält man die Verbindung der Formel

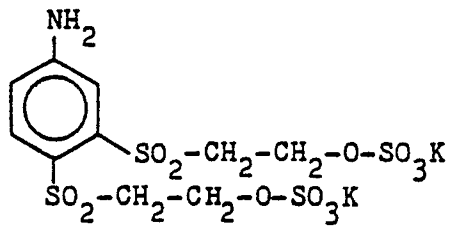

in vergleichbarer Ausbeute und Qualität.

Beispiel 11

Es wird eine Mischung aus 30,9 Teilen der Verbindung der Formel

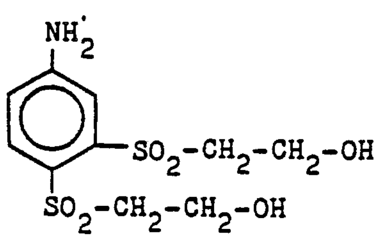

40 Volumenteilen Essigsäure, 80 Teilen Wasser und 28 Volumenteilen konzentrierter Salzsäure hergestellt. Zu dieser Mischung gibt man bei 0-2 °C langsam 22 Volumenteile 5n-Natriumnitrit-Lösung und rührt bei dieser Temperatur bis zur vollständigen Diazotierung. Das überschüssige Nitrit wird mittels Sulfanilsäure zerstört. Die angefallene Diazoniumsalzsuspension gibt man nun bei 0-5 °C zu einer Lösung von 21,5 Teilen N-(Bis-oxethyl)-3-chloranilin in 160 Teilen Wasser und 35 Volumenteilen konzentrierter Salzsäure. Man hält das Kupplungsgemisch während 2 Stunden bei 0-5 °C und pH 2-2,5. Dann stellt man mit wäßriger Natronlauge auf pH 4 und filtriert das ausgefallene Produkt ab. Nach Waschen mit Wasser und Trocknen im Vakuum bei 70-80 °C erhält man 46 Teile des Produkts der Formel

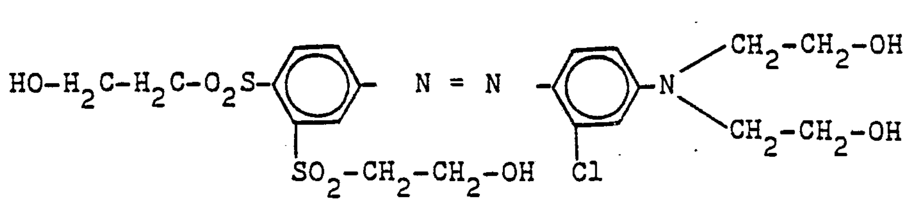

Zur Überführung in den Schwefelsäurehalbester trägt man 46 Teile dieser Verbindung bei 5-10 °C in 360 Volumenteile Schwefelsäuremonohydrat ein und rührt anschließend bei 20-25 °C 12 Stunden nach.

Dann gießt man die Reaktionsmischung auf 2000 Teile Eis/Wasser und neutralisiert bei maximal 10 °C mit Calciumcarbonat. Der ausgefallene Gips wird abgesaugt und die Farbstofflösung mit 20 Gew.-% Kaliumchlorid versetzt. Dabei scheidet sich der Farbstoff der Formel

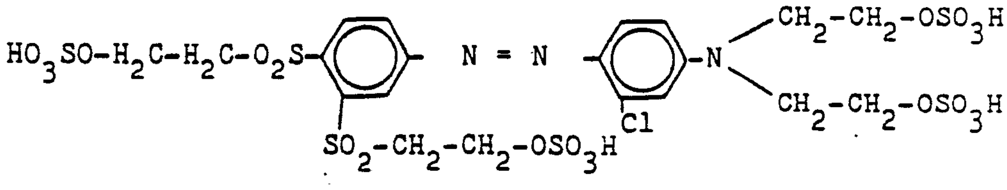

ab, der als Kaliumsalz vorliegt. Nach Trocknung im Vakuum erhält man 50 Teile eines roten Pulvers, das noch Kaliumchlorid enthält.

Der Farbstoff eignet sich sehr gut zum Färben und Bedrucken von Cellulosefasermaterialien, wie Baumwolle, und ergibt nach den für Reaktivfarbstoffe üblichen Applikations- und Fixiermethoden rote Färbungen und Drucke von guten Echtheitseigenschaften, insbesondere Naßechtheiten, wie guten Waschechtheiten bei 60 bis 95 °C.

Beispiel 12

61,8 Teile 2,5-Di-(β-hydroxyethylsulfonyl)-anilin werden bet etwa 20 °C in eine Mischung aus 200 Teilen Schwefelsäuremonohydrat und 25 Teilen 65 %igem Oleum unter Rühren eingetragen ; es wird noch 12 Stunden nachgerührt. Die erhaltene klare Lösung wird anschließend bei einer Temperatur unterhalb 5 °C in 1000 Teile einer 25 %igen wäßrigen Kaliumchlorid-Lösung langsam eingerührt. Man rührt noch drei Stunden nach und saugt das ausgefallene Produkt ab, wäscht es mit 25 %iger wäßriger Kaliumchlorid-Lösung und trocknet es. Es wird ein kaliumchloridhaltiges Pulver erhalten, welches das 2,5-Di-(ß-sulfatoethylsulfonyl)-anilin zu etwa 60 % enthält.

Eine Lösung von 45,9 Teilen (bezogen auf 100 %ige Substanz) der Kaliumverbindung des 2,5-Di-(ß-sulfatoethylsulfonyl)-anilins in 400 Teilen Wasser wird mit 200 Teilen Eis und 5 Volumenteilen 96 %iger Schwefelsäure versetzt. Man diazotiert durch langsame Zugabe von 10,2 Volumenteilen einer wäßrigen 5n-Natriumnitrit-Lösung innerhalb von 60 Minuten. Der Ansatz wird noch bei einer Temperatur zwischen 0 und 3 °C bis zur vollständigen Diazotierung nachgerührt, und schließlich der noch geringe Nitritüberschuß, wie üblich, mit Amidosulfonsäure zerstört.

Zu dieser Diazoniumsalzlösung läßt man unter Rühren innerhalb 10 Minuten bei etwa 5 °C eine wäßrige schwefelsaure Lösung aus 18,8 Teilen 3-Chlor-N,N-bis-(β-sulfatoethyl)-anilin einlaufen. Man stellt mit Natriumcarbonat oder vorzugsweise zur Bindung der Schwefelsäure und deren Ausfällung als Calciumsulfat mit Calciumcarbonat einen pH-Wert zwischen 2 und 2,2 ein und hält das Kupplungsgemisch noch 3 Stunden bei diesem pH-Wert. Sodann wird mit Natriumcarbonat oder vorzugsweise Calciumcarbonat ein pH-Wert von 5,5 eingestellt und das ausgefallene Calciumsulfat abgesaugt. Das Filtrat wird mit Kaliumchlorid in einer Menge, die 20 % des Volumens des Filtrates entspricht, unter Rühren versetzt ; es wird 4 Stunden nachgerührt und das ausgefallene Produkt abgesaugt und getrocknet.

Es wird ein organgerotes Pulver erhalten, das neben Kaliumchlorid den reaktiven Monoazofarbstoff der Formel

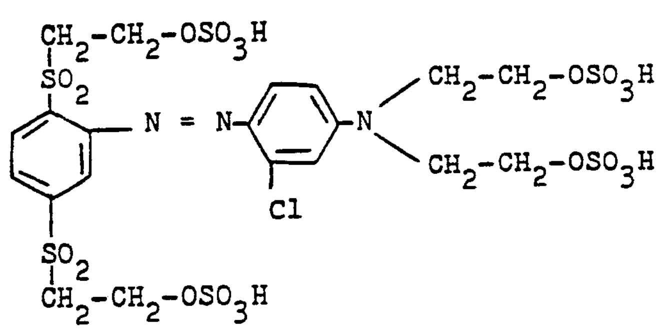

in Form des kaliumsalzes enthält. Dieser Farbstoff, der in wäßriger Lösung ein $\lambda_{max}$ von 479 nm zeigt, besitzt sehr gute Farbstoffeigenschaften und eignet sich sehr gut zum Färben und Bedrucken von Cellulosefasermaterialien, wie Baumwolle. Unter Anwendung der für faserreaktive Farbstoffe üblichen Applikations- und Fixiermethoden liefert er mit hohem Fixiergrad farbstarke orangerote Färbungen und Drucke mit guten Echtheitseigenschaften, insbesondere Naßechtheiten, wie insbesondere guten Waschechtheiten bei 60 bis 95 °C, guter alkalischer Schweißechtheit und Chlorwasserechtheit sowie guten Schweißlichtechtheiten.

## Patentansprüche

1. Monocyclische Bis-oxethylsulfonyl-aniline der allgemeinen Formel (1)

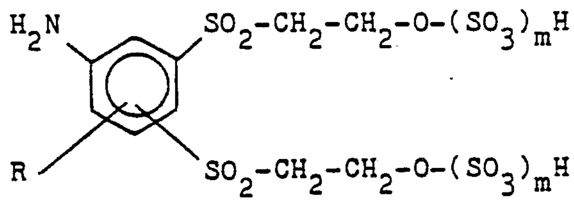

(1)

in welcher R ein Wasserstoff-, Chlor- oder Bromatom oder eine $\text{Alkyl}_{C_1\text{-}C_4}$- oder $\text{Alkoxy}_{C_1\text{-}C_4}$-Gruppe bedeutet und m die Zahl 0 oder 1 darstellt, und in welcher die Oxethylsulfonyl(schwefelsäurehalbester)-Gruppen in o- oder p-Stellung zueinander stehen.

2. Verbindung der Formel

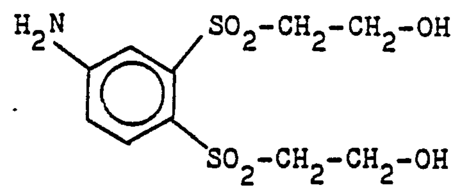

3. Verbindung der Formel

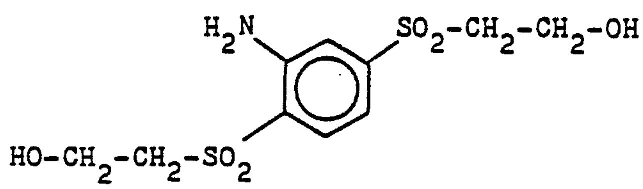

4. Verbindung der Formel

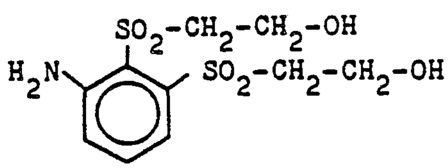

5. Verbindung der Formel

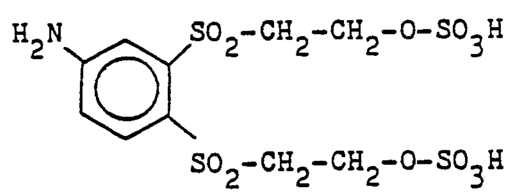

6. Verbindung der Formel

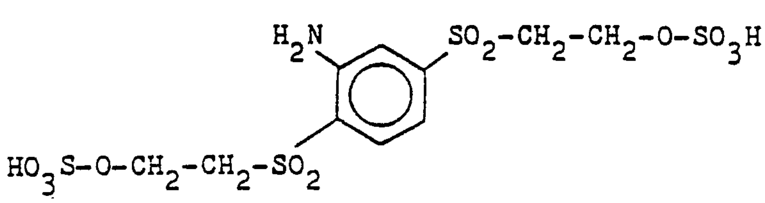

7. Verbindung der Formel

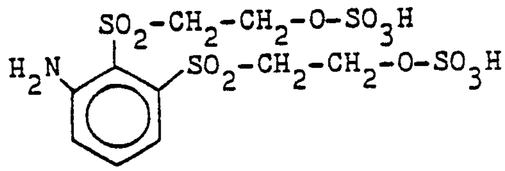

8. Verfahren zur Herstellung von monocyclischen Bis-oxethylsulfonyl-anilinen der allgemeinen Formel (1)

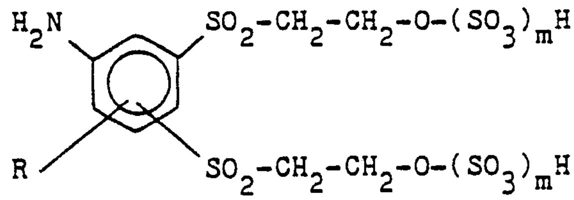

(1)

in welcher R ein Wasserstoff-, Chlor- oder Bromatom oder eine Alkyl$_{C_1-C_4}$- oder Alkoxy$_{C_1-C_4}$-Gruppe bedeutet, und m die Zahl 0 oder 1 darstellt, und in welcher die Oxethylsulfonyl(schwefelsäurehalbester)-Gruppen in o- oder p-Stellung zueinander stehen, dadurch gekennzeichnet, daß man eine Verbindung der Formel (2)

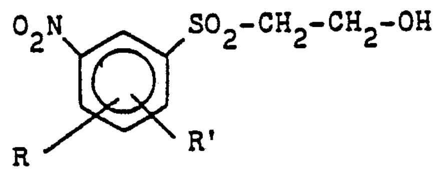

(2)

in welcher R die vorstehend genannte Bedeutung hat, R' ein Chlor- oder Bromatom bedeutet und in o- oder p-Stellung zur β-Oxethylsulfonyl-Gruppe steht, gegebenenfalls unter Zusatz von Wasser als Verdünnungsmittel, mit der mindestens stöchiometrischen Menge Thioglykol in Gegenwart einer mindestens stöchiometrischen Menge einer säurebindenden Alkalimetall- oder Erdalkalimetallverbindung bei Temperaturen von 10-60 °C, vorzugsweise 20-40 °C, umsetzt, die so erhaltene Verbindung der Formel (3)

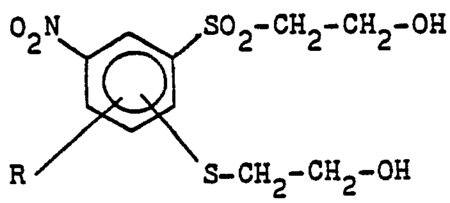

(3)

in welcher R die vorstehend genannte Bedeutung hat und die beiden schwefelhaltigen Substituenten in o- oder p-Stellung zueinander stehen, ohne oder nach erfolgter Zwischenisolierung, nach Zugabe katalytischer Mengen von Wolframtrioxid oder Alkaliwolframat und Wasser und Einstellung eines pH-Werts von 4-6 durch Zugabe einer nichtoxydierenden anorganischen oder organischen Säure, mittels Wasserstoffperoxid bei Temperaturen von 40 °C bis 100 °C oxidiert und die hierbei erhaltene Verbindung der Formel (4)

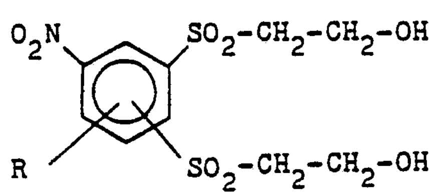

(4)

in welcher R die vorstehend genannte Bedeutung hat und die beiden β-Oxethylsulfonyl-Gruppen in o- oder p- Stellung zueinander stehen, in an sich bekannter Weise mittels Eisen/Säure oder katalytisch mit Nickel- oder Edelmetallkatalysatoren in wäßrigem Medium reduziert und die hierbei erhaltenen Verbindungen der allgemeinen Formel (5)

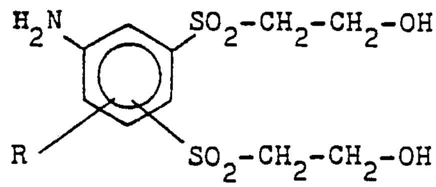

(5)

in welcher R die vorstehend genannte Bedeutung hat und die beiden β-Oxethylsulfonyl-Gruppen in o- oder p-Stellung zueinanderstehen, gegebenenfalls in an sich bekannter Weise mit konzentrierter Schwefelsäure, Oleum, einer Mischung aus Schwefelsäure-Monohydrat und Oleum, Schwefeltrioxid oder Chlorsulfonsäure in den Bisschwefelsäurehalbester der genannten Formel (1) mit m = 1 überführt.

## Claims

1. A monocyclic bisoxethylsulfonylaniline of the formula (1)

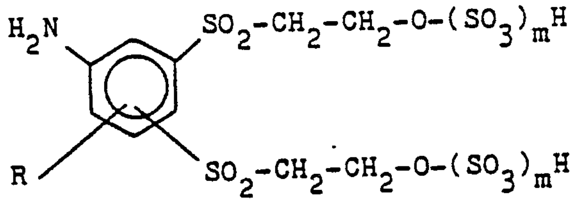

(1)

in which R denotes a hydrogen, chlorine or bromine atom or a $C_1$-$C_4$-alkyl or $C_1$-$C_4$-alkoxy group and m represent the number 0 or 1, and in which the oxethylsulfonyl (sulfuric acid halfester) groups are in the o-position or p-position in relation to one another.

2. The compound of the formula

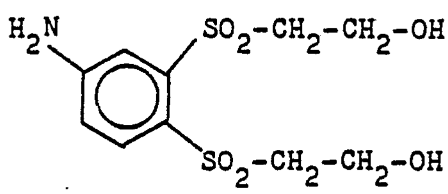

3. The compound of the formula

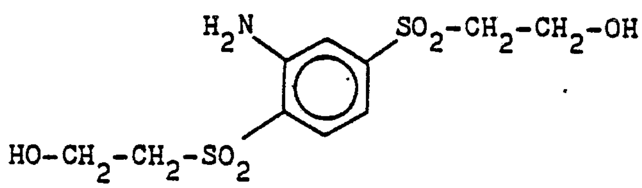

4. The compound of the formula

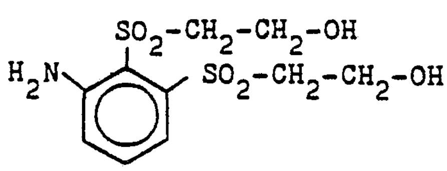

5. The compound of the formula

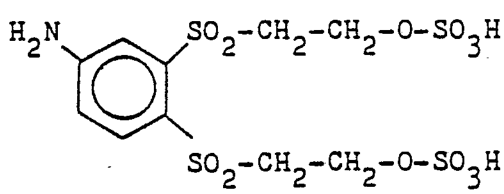

6. The compound of the formula

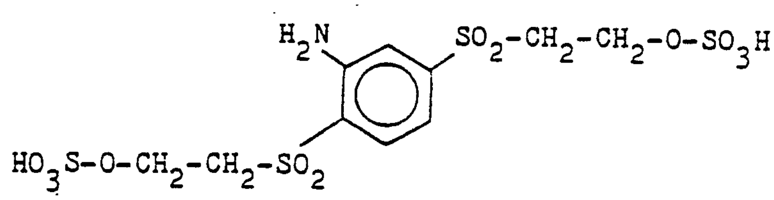

7. The compound of the formula

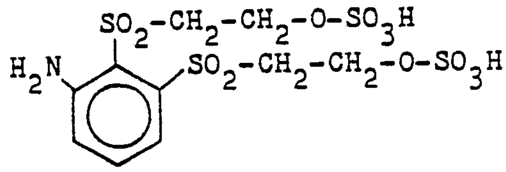

8. A process for the preparation of a monocyclic bisoxethylsulfonylaniline of the formula (1)

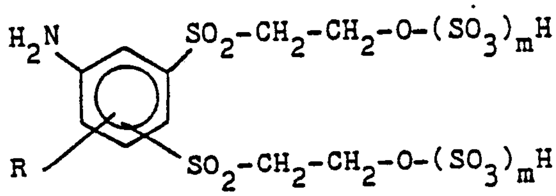

(1)

in which R represents a hydrogen, chlorine or bromine atom or a $C_1$-$C_4$-alkyl or $C_1$-$C_4$-alkoxy group and m represents the number 0 or 1, and in which the oxethyl sulfonyl(sulfuric acid halfester) groups are in the o-position or p-position in relation to one another, which comprises reacting a compound of the formula (2)

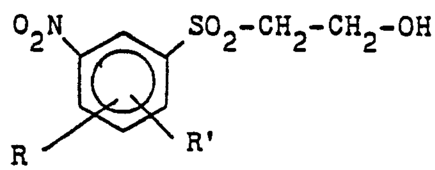

(2)

in which R has the meaning mentioned above, R′ denotes a chlorine or bromine atom and in the o-position or p-position relative to the (β)-oxethylsulfonyl group, if appropriate with the addition of water as a diluent, with at least the stoichiometric amount of thioglycol in the presence of an at least stoichiometric amount of an acid-binding alkali metal or alkaline earth metal compound at temperatures of 10-60 °C, preferably 20-40 °C oxidizing, by means of hydrogen peroxide at temperatures of 40 °C to 100 °C, the compound of the formula (3)

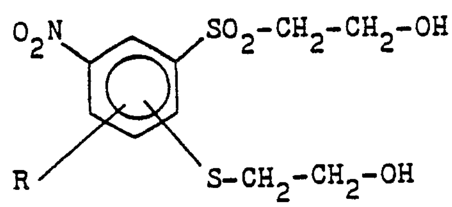

(3)

obtained in this way, in which R has the meaning mentioned above and the two sulfur-containing substituents are in the o-position or p-position in relation to one another, with or without intermediate isolation and after the addition of catalytic amounts of tungsten trioxide or an alkali metal tungstate and water, the pH being adjusted to a value of 4-6 by adding a non-oxidizing inorganic or organic acid, and reducing, in a manner known per se, by means of iron/acid or by catalytic means using nickel or noble metal catalysts in an aqueous medium, the compound of the formula (4)

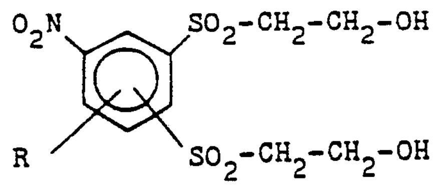

(4)

obtained in this way, in which R has the meaning mentioned above and the two (β)-oxethylsulfonyl groups are in the o-position or p-position in relation to one another, and converting the compounds of the formula (5)

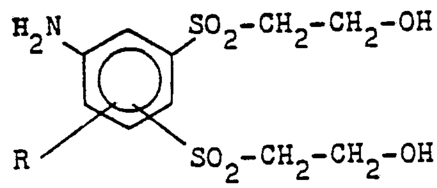

(5)

obtained in this way, in which R has the meaning mentioned above and the two β-oxethylsulfonyl groups are in the o-position or p-position in relation to one another, if appropriate in a manner known per se, by means of concentrated sulfuric acid, oleum, a mixture of sulfuric acid monohydrate and oleum, sulfur trioxide or chlorosulfonic acid into the bis-sulfuric acid half-ester of the formula (1) mentioned in which m = 1.

**Revendications**

1. Bis-(oxyéthylsulfonyl-anilines monocycliques qui répondent à la formule générale 1 :

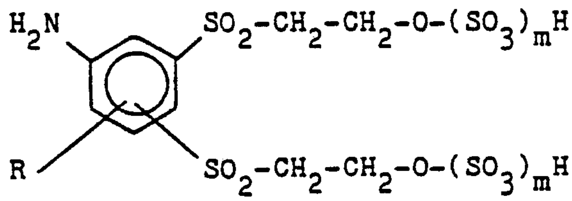

(1)

dans laquelle R représente un atome d'hydrogène, de chlore ou de brome, un radical alkyle en $C_1$-$C_4$ ou un radical alcoxy en $C_1$-$C_4$, et m représente le nombre 0 ou le nombre 1, et dans laquelle les radicaux hydroxy-éthylsulfonyles ou sulfatoéthylsulfonyles sont en position ortho ou para l'un par rapport à l'autre.

2. Composé de formule :

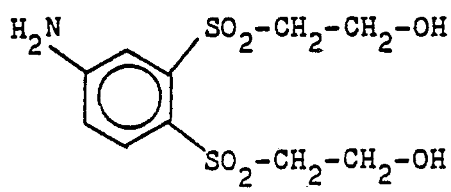

3. Composé de formule :

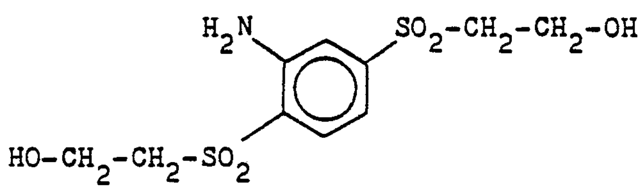

4. Composé de formule :

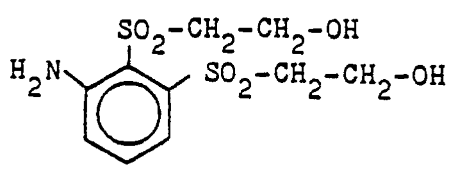

5. Composé de formule :

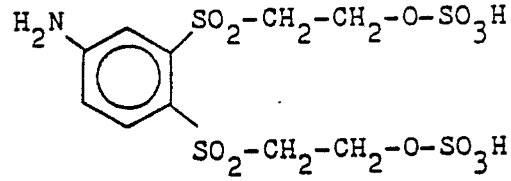

6. Composé de formule :

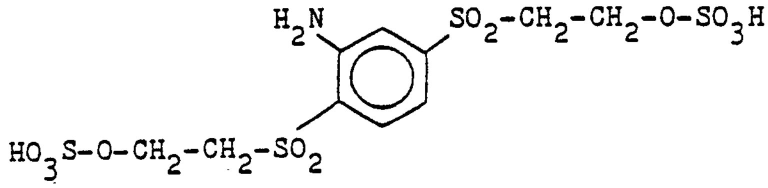

7. Composé de formule :

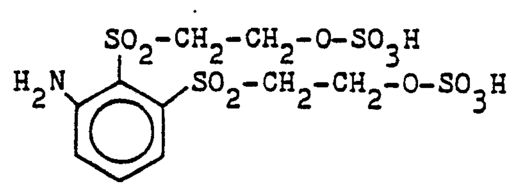

8. Procédé pour préparer des bis-(oxyéthylsulfonyl)-anilines monocycliques répondant à la formule générale 1 :

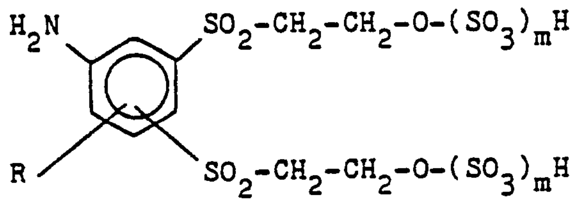

(1)

dans laquelle R représente un atome d'hydrogène, de chlore ou de brome ou un radical alkyle en $C_1$-$C_4$ ou alcoxy en $C_1$-$C_4$, et m représente le nombre 0 ou le nombre 1, et dans laquelle les radicaux hydroxyéthylsulfonyles ou sulfatoéthylsulfonyles se trouvent en position ortho ou para l'un par rapport à l'autre, procédé caractérisé en ce qu'on fait réagir un composé répondant à la formule 2 :

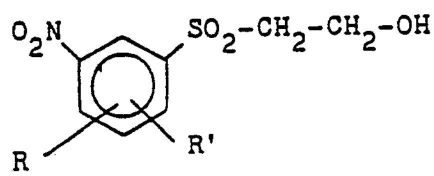

(2)

dans laquelle R a la signification qui lui a été donnée ci-dessus, R' représente un atome de chlore ou de brome et se trouve en ortho ou para relativement au radical oxy-2 éthylsulfonyle, éventuellement par addition d'eau comme diluant, avec une quantité de thioglycol au moins égale à la quantité stoechiométrique, en présence d'une quantité au moins égale à la quantité stoechiométrique d'un composé de métal alcalin ou de métal alcalino-terreux servant d'accepteur d'acides, à des températures de 10 à 60 °C, de préférence de 20 à 40 °C, puis, sans l'avoir isolé ou après l'avoir isolé intermédiairement, on oxyde le composé ainsi obtenu, qui répond à la formule 3 :

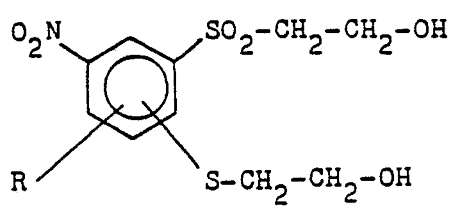

(3)

dans laquelle R a la signification qui lui a été donnée ci-dessus et les deux substituants sulfurés sont en position ortho ou para l'un par rapport à l'autre, après avoir ajouté des quantités catalytiques de trioxyde de tungstène ou d'un tungstate d'un métal alcalin et de l'eau, et avoir réglé le pH à 4-6 par addition d'un acide minéral ou organique non oxydant, au moyen de peroxyde d'hydrogène, à des températures de 40 à 100 °C, on réduit le composé ainsi obtenu, qui répond à la formule 4 :

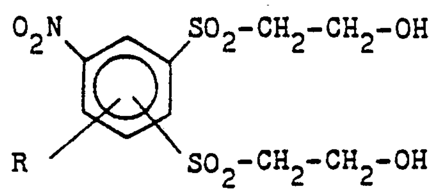

(4)

dans laquelle R a la signification qui lui a été donnée ci-dessus et les deux radicaux oxy-2 éthylsulfonyles sont en position ortho ou para l'un par rapport à l'autre, de manière connue, en milieu aqueux, au moyen de fer et d'un acide ou catalytiquement en présence de catalyseurs à base de nickel ou de métaux nobles, et on transforme éventuellement les composés ainsi obtenus, qui répondent à la formule générale 5 :

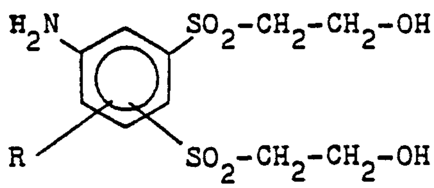

(5)

dans laquelle R a la signification qui lui a été donnée ci-dessus et les deux radicaux oxy-2 éthylsulfonyles se trouvent en position ortho ou para l'un par rapport à l'autre, de manière connue, au moyen d'acide sulfurique concentré, d'oléum, d'un mélange de monohydrate d'acide sulfurique et d'oléum, de trioxyde de soufre ou d'acide chlorosulfonique, en les bis-(hémi-esters sulfuriques) de formule 1 dans lesquels m est égal à 1.